(19) Europäisches Patentamt
European Patent Office
Office européen des brevets

(11) Publication number: 0 201 957 A2

# (12) EUROPEAN PATENT APPLICATION

(21) Application number: 86200682.2

(22) Date of filing: 21.04.86

(51) Int. Cl.⁴: C07C 51/41 , C07C 53/124 , C07C 59/08 , C07C 103/38 , C07D 205/04

(30) Priority: 14.05.85 GB 8512230

(43) Date of publication of application: 20.11.86 Bulletin 86/47

(84) Designated Contracting States: AT BE CH DE FR GB IT LI LU NL SE

(71) Applicant: SHELL INTERNATIONALE RESEARCH MAATSCHAPPIJ B.V.
Carel van Bylandtlaan 30
NL-2596 HR Den Haag(NL)

(72) Inventor: Van Helden, Robert
Badhuisweg 3
NL-1031 CM Amsterdam(NL)

(74) Representative: Hunter, Keith Roger Ian et al
4 York Road
London SE1 7NA(GB)

## (54) Process for the preparation of a carboxylic acid salt.

(57) A carboxylic acid salt is prepared by contacting a compound containing at least one primary alcohol group with a platinum containing catalyst at elevated temperature in an aqueous solution, containing at least one alkali metal and/or alkaline earth metal hydroxide.

EP 0 201 957 A2

# PROCESS FOR THE PREPARATION OF A CARBOXYLIC ACID SALT

The invention relates to a process for the preparation of a carboxylic acid salt from a primary alcohol.

From United States patent specification No. 3,708,533 it is known to prepare amino carboxylic acid salts from amino alcohols by contacting said alcohols with alkali metal hydroxides over a catalyst comprising a Group IIB or Group VIII metal. The preferred catalysts include cadmium, zinc, palladium or nickel. Cadmium oxide is particularly preferred. The known preparation method is carried out at a pressure between about 9.5 and 122 bar and at a temperature ranging from 199 to 288 °C.

The known method can thus only be applied to amino alcohols, and moreover an elevated pressure and a relatively high temperature are required.

Surprisingly, it has now been found that use of a platinum-containing catalyst under alkaline conditions enables many primary alcohols, not only amino alcohols, to be converted to carboxylic acid salts. Further, use of a platinum-containing catalyst instead of a catalyst containing any other Group VIII metal, enables the conversion to be carried out at pressures and temperatures lower than those prevailing in the known preparation method.

The invention therefore relates to a process for the preparation of a carboxylic acid salt in which a compound having at least one primary alcohol group is contacted with a platinum-containing catalyst at elevated temperature in an aqueous solution, containing at least one alkali metal and/or alkaline earth metal hydroxide.

The primary alcohol group-containing compounds used as starting material in the process according to the present invention include organic compounds containing from 1 to 20 carbon atoms, substituted with one or more hydroxyl groups. Examples of suitable alcohols are methanol, ethanol, n-propanol, n-butanol, n-pentanol, ethylene glycol and propylene glycol. Amino alcohols, such as triethanol amine are also very suitable as starting material. When the platinum catalyst is applied to secondary alcohols no reaction occurs, although in some cases the formation of ketones takes place which can cause side-reactions.

The catalyst used in the present process may consist of platinum alone. However, such a catalyst would not only be extremely expensive but it would also have the drawback that it would have a relatively low catalytically active specific area. This latter drawback could be obviated by augmenting the platinum surface, e.g. by using so-called platinum whiskers, i.e. metallic filamentary crystals, like those described in British patent specification No. 703,460. It is, however, preferred to use in the process according to the present invention a catalyst which comprises platinum on a carrier. The carrier may be one of those materials conventionally used as catalyst supports although, of course, it should be substantially inert to the alkaline conditions employed. Preferably, the carrier is carbon. The high specific area of active carbon gives a high activity of the catalyst. The specific area of active carbon is suitably from 40 to 400 $m^2/g$. The platinum content of a carrier-containing catalyst can vary within wide ranges. At a platinum content below 0.1 %w, calculated on the total of platinum and carrier, the catalyst activity will become too low to be practical whereas a platinum content over 20 %w would yield expensive catalysts without substantial activity increase. Therefore, the catalyst preferably contains from 0.1 to 20 %w of platinum, calculated on the total of platinum and carrier. Particularly preferred are catalysts comprising from 0.5 to 15 %w of platinum.

The amount of catalyst used may vary widely, and even traces of catalyst exert a discernible effect on the reaction. As larger amounts are used, the rate of reaction becomes more rapid, so that the upper limit on the amount of catalyst is fixed more by economic than by chemical considerations. For most practical purposes, the range of catalyst proportion is from 0.05 to 5 %w of platinum on the primary alcohol group-containing compound.

The activity of the platinum catalyst is indicated by the preferred reaction conditions. Whereas the reaction temperature of the prior art process must be over 199 °C, the reaction temperature in the process according to the invention can be as low as 80 °C, and is preferably from 90 to 170 °C. Conveniently, a temperature within these ranges can be obtained by heating the reaction mixture to its reflux temperature. The process is preferably carried out without the application of external pressure. The pressure can be as low as atmospheric. It is, of course, possible to carry out the process at elevated pressures, e.g. up to 50 bar. However, this would unnecessarily increase the capital expenditure for the process, for more expensive pressure vessels would be required for the reaction mixture. The possibility to operate in the present process at temperatures below 199 °C and at about atmospheric pressure represents a considerable advantage over the prior art process.

During the preparation of the carboxylic acid salt hydrogen is evolved. It is desirable to avoid reactions of the hydrogen evolved with oxygen in the air. Therefore, the process according to the invention is preferably carried out in an oxygen-free atmosphere. It is thus possible to substitute the air-containing atmosphere above the reaction mixture for an inert atmosphere, e.g. hydrogen, nitrogen or argon. From an economical point of view the reaction mixture is advantageously brought into a nitrogen atmosphere, the nitrogen being replaced during the reaction by hydrogen evolving from the reaction mixture.

The preparation according to the invention is carried out in an aqueous solution of a hydroxide. Use of a basic salt, e.g. the carbonate salt, instead of the hydroxide results in a little and slow conversion only, if any. The hydroxide used can be an alkali and/or alkaline earth metal hydroxide. Preferred are alkali metal hydroxides, in particular sodium and potassium hydroxide. These hydroxides are very cheap and excellently soluble in water, even in high concentrations. The concentration of the metal hydroxide is preferably at least 15 %w, calculated on the total of water and the metal hydroxide. Generally, from practical point of view the concentration of the metal hydroxide does not exceed 80 %w although higher concentrations can be used. It is even possible to carry out the reaction with a metal hydroxide alone, yielding first metal alcoholate and water, thereby creating an aqueous solution comprising water, alcoholate and hydroxide. Subsequently the oxidation to the carboxylic acid salt takes place. In this case, however, to avoid the reaction mixture from becoming solid, an excess of the primary alcohol is to be used, which requires additional separation steps to recover the excess of alcohol.

The amount of the hydroxide is suitably such that the molar ratio of the hydroxyl ions to the hydroxymethyl groups in the primary alcohol group-containing compounds ranges from 1:1 to 15:1.

The carboxylic acid salts produced by the present process may, when required, be converted into the corresponding carboxylic acids by treating them with an aqueous acid solution or by ion exchange. Suitable aqueous acid solutions include aqueous solutions of strong inorganic acids, such as sulphuric, nitric, hydrochloric, hydrobromic acid, or of organic acids such as formic acid or chloroacetic acid. It is also possible to subject the salt to ion exchange using a suitable ion exchange resin as known in the art.

When desired, one can use the salts produced directly for further reactions or for the synthesis of e.g. esters.

It has been stated hereinbefore that many primary alcohols can be used in the process according to the present invention. Excellently suitable as starting material are triethanolamine and 3,3-bis-(hydroxymethyl) azetidine derivatives. The reaction product of triethanolamine is nitrilo triacetic acid or salt thereof, and these products are advantageous substitutes for phosphate in laundering compositions.

Other suitable starting materials for the process according to the invention are 3,3-bis-(hydroxymethyl)azetidine derivatives of the formula

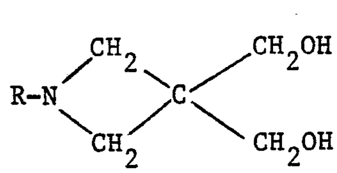

in which R is hydrogen or a protective group such as $C_1$-$C_{18}$ alkyl, optionally substituted aryl, benzyl, diphenylmethyl, tosyl, etc. These starting materials may be easily converted by the present process into azetidine-3,3-dicarboxylic acid or -3-monocarboxylic acid derivatives of the formulae

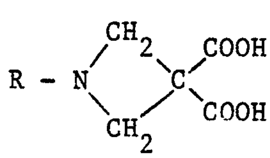

and

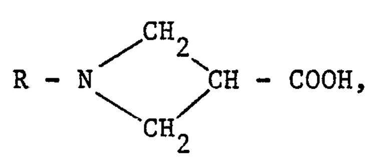

respectively.

Azetidine-3-carboxylic acid is a plant hybridising agent, its mode of action presumably being based on its ability to produce male sterility in plants. The direct product when the present process is applied to 3,3-bis(hydroxymethyl)azetidine derivates is the dibasic salt of the corresponding azetidine-3,3-dicarboxylic acid. It is possible to isolate either the dibasic salt or the dicarboxylic acid and subject the isolated product to decarboxylation. The decarboxylation be be carried out with or without solvents, though a solvent is preferably used. The solvent can either be made acidic or alkaline. When an acid medium is used, it suitably comprises an aqueous solution of a strong mineral acid (e.g. sulphuric, hydrochloric, hydrobromic, nitric acid) or of an organic acid such as formic, or acetic acid. Suitable alkaline solutions include aqueous solutions of metal hydroxides or carbonates. When the decarboxylation is carried out without a solvent, it is desirable to add a base, such as amines, or pyridine. Acceleration of the decarboxylation reaction can frequently be achieved by the addition of copper or copper(I) salts. The decarboxylation can be carried out at elevated temperatures, suitably between 80 and 230 °C.

The invention therefore also relates to a process for the preparation of azetidine-3-carboxylic acid or a salt thereof, optionally having a substituent attached to the nitrogen atom of the azetidine ring, in which the corresponding 3,3-bis-(hydroxymethyl) azetidine derivative is subjected to the conversion of the present invention, and the product thereof is subjected to decarboxylation.

It is preferred to prepare azetidine-3-carboxylic acid derivatives directly from the azetidine-3,3-bis-(hydroxymethyl) derivates, i.e. without isolation of the dibasic salt or of the dicarboxylic acid compound. Therefore, derivates of azetidine-3,3-dicarboxylic acid or dibasic salts thereof are conveniently prepared by the process according to the present invention, and subsequently decarboxylated by prolonging the heating at a temperature between 80 and 200 °C, in particular at the reflux temperature.

The substituents of the nitrogen atoms can be selected from e.g. $C_1$-$C_{18}$ alkyl, optionally substituted aryl, benzyl or diphenylmethyl, and tosyl. However, it is preferred to use unsubstituted azetidine-3,3-bis(hydroxymethyl), since this yields the mono-carboxylic acid in excellent yield.

The invention will further be illustrated by means of the following Examples.

## EXAMPLES

In the following experiments the reactants were placed into a polytetrafluoroethylene-lined reactor provided with a magnetic stirrer and a reflux condensor. The reactor was heated to about 100 °C in a nitrogen atmosphere, and subsequently the catalyst was added. The resulting mixture was heated to reflux temperature (110-120 °C). The heating was stopped when practically no hydrogen evolved anymore. The reaction product was diluted with water, and the catalyst was removed by filtration, washed with water and dried. From the filtrate water was removed by distillation. The composition of the resulting residue was determined by NMR.

### EXAMPLE 1

11.1 g n-Butyl alcohol, 8.0 g sodium hydroxide, 15.0 g water were mixed as described above. The 2.4 g of a 5 %w Pt on carbon catalyst (1 %w Pt on n-butyl alcohol) was added to the mixture. After 22 h the reaction mixture was treated as described above, to yield 18.2 g practically pure sodium butyrate. After acidification with hydrochloric acid and extraction with diethylether 10.0 g pure n-butyric acid was obtained (yield 68%)

### EXAMPLE 2

Under the same conditions as described in Example 1 13.8 g glycerine, 6.0 g sodium hydroxide and 11.0 g water reacted in the presence of 2.8 g of a 5 %w Pt on carbon catalyst (1 %w Pt on glycerine). After 7 h hydrogen formation stopped. Working up in the usual way gave 14.5 g solid residue consisting of the sodium salt of lactic acid - (96 %m on intake of glycerine).

### EXAMPLE 3

A similar experiment as in Example 2 was carried out starting from 11.4 g 1,2-propylene glycol, 6.0 g sodium hydroxide, 11.0 g water and 2.4 g of a 5 %w Pt on carbon catalyst (1 %w Pt on propylene glycol). After 7½ h hydrogen formation nearly stopped. Working up yielded 15.0 g of a residue. NMR analysis showed a propylene glycol conversion of 90% with a selectivity to lactic acid of 89%. In addition acetic acid (selectivity 7%) and higher aliphatic acids (selectivity 4%) were obtained.

EXAMPLE 4

Five other experiments were run using propylene glycol as starting material in which the amount of the catalyst was varied, the reuse of recovered catalyst was tested and the amount and concentration of sodium hydroxide was alterated. The results of these experiments are shown in Table I.

TABLE I

Temp. 110-120 °C, start under nitrogen, 5 %w Pt on carbon catalyst. Propylene glycol (A) intake 11.4 g.

| Exp. No. | aq NaOH %w | NaOH/A molar ratio | Pt %m on A | time h | conv. %m | selectivity lactic acid %m | acetic acid %m | higher** aliphatic acids %m |
|---|---|---|---|---|---|---|---|---|
| 1 | 35 | 1 | 0.43 | 7¼ | 90 | 83 | 11 | 6 |
| 2 | 35 | 1 | 1.2 | 7½ | 90 | 89 | 7 | 4 |
| 3 | 35 | 1 | 0.4* | 8¾ | 91 | 80 | 10 | 10 |
| 4 | 52 | 2 | 0.4 | 4 | 96 | 79 | 4 | 17 |
| 5 | 52 | 2 | 0.1 | 4 | 96 | 79 | 6 | 15 |

* catalyst reused, recovered from (1)

** mol weight 138-170

Comparison of the results of experiments 1 and 2 or 4 and 5 shows that using three or four times the amount of catalyst does not increase the reactivity. Comparison of the results of experiments 1 and 3 shows that the catalyst hardly loses any activity when it is reused and from experiments 1 and 4 it appears that increasing the hydroxide concentration results in a faster reaction.

EXAMPLE 5

Similar experiments as described in Example 1 were carried out using triethanolamine (1) as starting material. The product obtained included the sodium salt of nitrilotriacetic acid (2) and of ethanol amine-diacetic acid (3). The results and conditions are indicated in Table II.

TABLE II

| Exp. No. | NaOH g | 1 g | water g | 5 %w Pt/C g | time h | conv. %m | selectivity (%m) 2 | 3 |
|---|---|---|---|---|---|---|---|---|
| 1 | 10.0 | 11.0 | 14.0 | 4.4 | 21 | 100 | 60 | 40 |
| 2 | 10.0 | 11.0 | 10.0 | 0.4 | 20 | 100 | 62 | 38 |

Comparative Experiments

An experiment as described in Example 1 was carried out using 11.0 g triethanolamine, 12.0 g water, 10.0 g sodium hydroxide and 1.0 g cadmium oxide. The mixture was reacted for 66 h at 110-120 °C. Hydrogen did not evolve. The reaction mixture was filtered to remove the white coloured cadmium oxide derived compounds (probably $Na_2Cd(OH)_4$). The filtrate was distilled to remove water yielding 22.7 g of a solid residue. NMR analysis indicated that less than 1 %m-N-$CH_2COOH$ groups were present and >99 %m -N-$CH_2$-$CH_2OH$ groups.

In another experiment 1.0 g of 5 %w Pd on carbon was tested in a mixture comprising 10.0 g triethanol amine, 12.0 g water and 10.0 g sodium hydroxide. After 23 h the following products were obtained: 40 %m unconverted triethanolamine and 60 %m diethanolaminemonoacetic acid sodium salt.

EXAMPLE 6

An experiment was carried out under the same conditions as in example 1 using 11.3 g 3,3-bis-(hydroxymethyl)azetidine

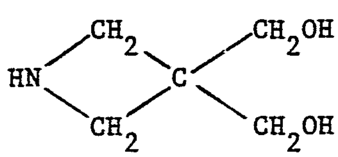
(I),

16.0 g water and 10.0 g sodium hydroxide. In a nitrogen atmosphere 7.5 g of a 5 %w Pt on carbon catalyst -containing 56 %w water -was added at 100 °C (1.5 %w Pt on intake I). The reaction mixture was heated to gentle reflux (110-120°C). Hydrogen is evolved, after 20 h the conversion of I is 100%, as shown by NMR analysis. The reaction product is diluted with water and the catalyst removed by filtration. From the aqueous filtrate water was removed by distillation, yielding 21.7 g solid white residue. NMR analysis indicated this residue to be a mixture of the sodium salt of azetidine -3-carboxylic acid II and sodium formate in a molar ratio of 1:1.

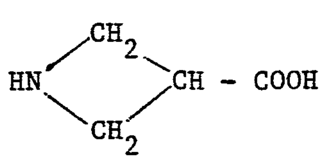
(II)

The residue was treated with 200 ml water and 50 ml concentrated hydrochloric acid and evaporated to dryness (bath temp. 90 °C at 1 mm Hg). The residue obtained was dissolved in water to a volume of 240 ml. 50 ml of this solution was passed over a Dowex 50W-X8 acidic anion exchange resin. After washing neutral with water the amino acid was eluted from the resin with 6% aqueous ammonia. The aqueous solution was evaporated to dryness (final bath temp. 80 °C at 1 mm Hg) and the residue analysed by NMR. This yielded 1.9 g crude azetidine-3-carboxylic acid (83 %m on intake I). Nearly pure product was obtained by treatment of the crude material with isopropylalcohol.

EXAMPLE 7

A similar experiment as described in Example 6 was carried out using 14.5 g crude N-ethyl-3,3-bis(hydroxymethyl)azetidine (III)

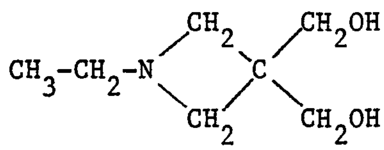

(III)

9.0 g sodiumhydroxide, 16.0 g water and 8 g of a 5 %w Pt on carbon catalyst containing 52.4 %w water. After 16 h hydrogen formation had nearly stopped. The reaction product was worked up in the usual way, yielding 20.4 g solid white material. NMR analysis indicated nearly complete conversion of III to N-ethyl azetidine carboxylic acid (IV)

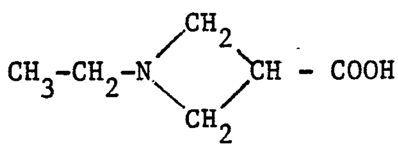

(IV)

The amino acid IV was isolated in the same way as described in Example 6 using Dowex 50W-X8 anion exchange resin (white crystalline solid 75 %m yield on intake III).

EXAMPLE 8

A similar experiment as in example 6 was carried out starting from 3.7 g N-benzyl-3,3-bis-(hydroxymethyl)azetidine

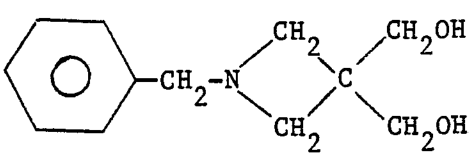

(V)

(V), 8.0 g sodium hydroxide, 15.0 g water and 2 g of a 5 %w Pt on carbon catalyst (2.7 %w Pt on intake V).

After 20 h the reaction product was diluted with water and the catalyst removed by filtration. This gave after drying 2 g recovered catalyst. The aqueous filtrate was extracted three times with diethylether. In the ether solution V could not be detected anymore (conversion 100%). From the aqueous solution water was removed and 14.6 g solid white residue was obtained (124 %w on intake V + NaOH) which according to NMR consisted mainly of the disodium salt of N-benzyl azetidine-3,3-dicarboxylic acid. This residue was refluxed with 200 ml ethylalcohol for 1 h and the insoluble white material collected by filtration and

dried (4.9 g). NMR analysis showed the presence of 71 %m of the diacid VI and 11 %m of N-benzyl azetidine-3-carboxylic acid VII on intake of the diol V.

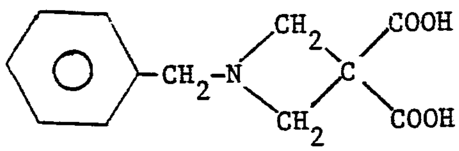

(VI)

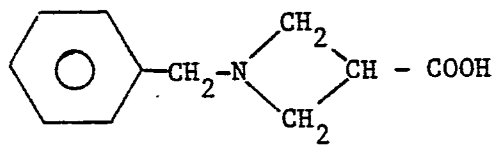

(VII)

The ethyl alcoholic filtrate was diluted with water and evaporated to dryness (80 °C at 20 mm Hg). The solid residue of 9.2 g was analysed with NMR. This showed the presence of 14 %m of the monocarboxylic acid VII.

Refluxing of the crude disodium salt of VI with excess formic acid for 24 h yielded the pure monocarboxylic acid VII. Upon neutralization of the crude disodium salt of VII with 4N HCl, the pure dicarboxylic acid VII was separated and collected by filtration.

## Claims

1.Process for the preparation of a carboxylic acid salt in which a compound containing at least one primary alcohol group is contacted with a platinum-containing catalyst at elevated temperature in an aqueous solution, containing at least one alkali metal and/or alkaline earth metal hydroxide.

2. Process according to claim 1, in which the catalyst comprises platinum on a carrier.

3. Process according to claim 2, in which the carrier is carbon.

4. Process according to claim 2 or 3, in which the catalyst contains from 0.1 to 20 %w of platinum, calculated on the total of platinum and carbon.

5. Process according to any one of claims 1-4, in which the temperature ranges from 90 to 170 °C.

6. Process according to any one of claims 1-5, in which the reaction is carried out without the application of external pressure.

7. Process according to any one of claims 1-6, which is carried out in an oxygen-free atmosphere.

8. Process according to any one of claims 1-7, in which the aqueous solution contains from 15 to 80 %w of metal hydroxide, calculated on the total of water and metal hydroxide.

9. Process according to any one of claims 1-8, in which the amount of metal hydroxide is such that the molar ratio of the hydroxyl ions to the hydroxymethyl groups in the primary alcohol group-containing compounds ranges from 1:1 to 15:1.

10. Process for the preparation of a carboxylic acid, in which the carboxylic acid salt produced according to any one of claims 1-9 is treated with an aqueous acid or is subjected to ion exchange.

11. Process for the preparation of nitrilotriacetic acid or a salt thereof in which triethanolamine is subjected to a process according to any one of claims 1-10.

12. Process for the preparation of azetidine-3-carboxylic acid or salt thereof, optionally having a substituent attached to the nitrogen atom of the azetidine ring, in which the corresponding 3,3-bis-(hydroxymethyl)azetidine derivative is subjected to a process according to any one of claims 1-10, and the product thereof is subjected to decarboxylation.

13. Process according to claim 13, in which the decarboxylation is carried out without isolation of intermediate products by prolonging the heating of the reaction mixture at a temperature between 80 and 200 °C.

14. Process according to claim 1, substantially as described hereinbefore with particular reference to the Examples.

15. A carboxylic acid derivative whenever prepared according to any one of claims 1-15.